(19) Europäisches Patentamt
European Patent Office
Office européen des brevets

(11) Publication number: **0 122 978**
**B1**

(12) **EUROPEAN PATENT SPECIFICATION**

(45) Date of publication of patent specification: **18.05.88**

(21) Application number: **83113242.8**

(22) Date of filing: **30.12.83**

(51) Int. Cl.⁴: **C 07 D 487/04,**
A 61 K 31/505, A 61 K 31/53
// (C07D487/04, 251:00, 231:00),(C07D487/04, 251:00, 249:00)

(54) **Therapeutically active di-, tri-, tetra- and penta-aza indenes.**

(30) Priority: **30.12.82 US 454734**

(43) Date of publication of application:
**31.10.84 Bulletin 84/44**

(45) Publication of the grant of the patent:
**18.05.88 Bulletin 88/20**

(84) Designated Contracting States:
**AT BE CH DE FR GB IT LI LU NL SE**

(56) References cited:
**GB-A-1 601 132**
**US-A-4 005 205**

(73) Proprietor: **BIOMEASURE INC.**
**11-15 E. Avenue**
**Hopkinton Massachusetts (US)**

(72) Inventor: **Kim, Sun Hyuk**
**20 Whitney Street**
**Chestnut Hill Massachusetts (US)**
Inventor: **Moreau, Jacques-Pierre**
**159 Westboro Road Upton**
**Massachusetts (US)**

(74) Representative: **Heidrich, Udo, Dr. jur., Dipl.-Phys.**
**Franziskanerstrasse 30**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 122 978 B1

**Description**

Background of the Invention

This invention relates to compounds that block gastric secretion.

Such compounds, which may be histamine $H_2$ receptor antagonists, reduce the volume and acidity of gastric secretions caused by a variety of stimuli, e.g., insulin, histamine, gastrin, food, and parasympathetic activity.

Summary of the Invention

In general, the invention features compounds having gastric secretion reducing activity and having the general formula

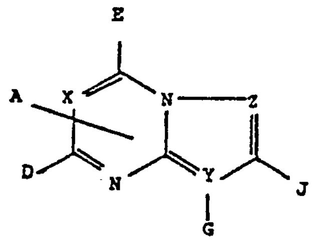

(1)

wherein D is H, SH, OH, $NH_2$, or $R^4S$ where $R^4$ is lower (fewer than 6 carbon atoms) alkyl; E is OH or $NH_2$; G is H or a halogen; J is H or an aryl group (having a single ring); X is CH or N; Y is CH or N or CT, wherein T is a halogen; Z is CH or N; and A replaces a hydrogen of either D or E and is chosen from

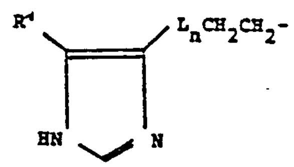

(2),

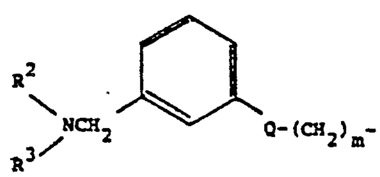

(3),

or

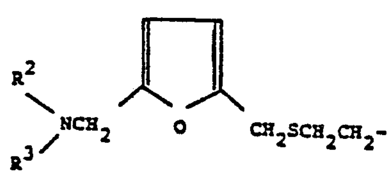

(4),

wherein $R^1$ is H or $CH_3$; L is $CH_2S$; Q is O or $CH_2S$; n is 0 or 1; $2 \leq m \leq 4$; each $R^2$ and $R^3$, independently, is hydrogen, lower alkyl, lower cycloalkyl, or lower aralkyl; or $R^2$ and $R^3$, together with the nitrogen atom to which they are attached, form a 4, 5, or 6 membered heterocyclic ring containing 0, 1, or 2 oxygen atoms and 1, 2, or 3 nitrogen atoms and being unsubstituted or lower alkyl substituted.

In preferred embodiments the compound is 2-(4-imidazolylethylamino)-4-oxo-1H,3H-pyrazolo[1,5-a]-1,3,5-triazine; 4-(2-(5-methyl-4-imidazolylmethylthio)-ethylamino)-2-methylthiopyrazolo[1,5-a]-1,3,5-triazine; 4-(4-imidazolylethylamino)-2-methylthiopyrazolo[1,5-a]-1,3,5-triazine; 8-bromo-4-(2-(5-methyl-4-imidazolylmethylthio)-ethylamino)-2-methylthiopyrazolo[1,5-a]-1,3,5-triazine; or 8-bromo-4-(4-imidazolylethylamino)-2-methylthiopyrazolo-[1,5-a]-1,3,5-triazine.

The compounds are potent, non-mutagenic, stable, and will pass through the stomach without losing their effectiveness. Furthermore, manufacture is relatively simple and inexpensive.

Other features and advantages of the invention will be apparent from the following description of the preferred embodiments, and from the claims.

Description of the Preferred Embodiments

*Structure*

The compounds of the invention have the general formula (1). Examples of preferred compounds are those referred to as preferred embodiments above.

The compounds are purine base derivatives having a nitrogen at the ring junction.

All the compounds can exhibit tautomerism, and the formulas are intended to cover all tautomers.

The compounds or pharmaceutically acceptable salts thereof can be administered alone or in combination with a pharmaceutically acceptable carrier or diluent.

Acceptable salts include hydrochlorides, hydrobromides, and sulfates. Particularly useful organic acid salts are acetates, maleates, and fumarates.

For oral administration the pharmaceutical composition can most conveniently be in the form of capsules or tablets, which may be slow release tablets. The composition can also be in the form of a dragee or syrup.

*Synthesis*

The above compounds can be synthesized as follows. A primary amine of Formula (5), (6), or (7)

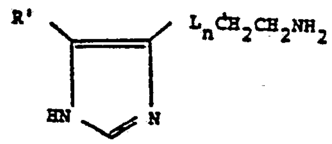

(5)

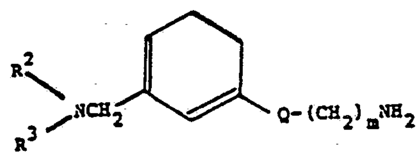

(6)

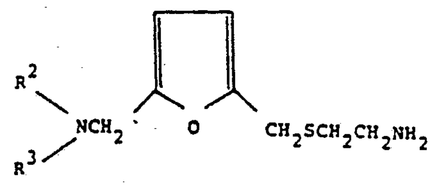

(7)

undergoes a condensation reaction with a reagent such as

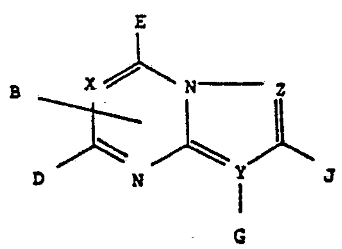

(8)

wherein B replaces either D or E and is a good leaving group, e.g., a halogen (e.g. Cl, Br), alkylthio, alkylsulfoxo, or alkylsulfono group. D, E, G, J, X, Y, and Z in formula (8) are as defined above for Formula (1).

The above reaction will occur either in a protic (e.g., water, alcohol, ethoxyethanol, or ethoxyethoxyethanol) or aprotic (e.g., toluene or xylene) solvent at temperatures from ambient to reflux. The amine can be in the form of the free base or in the form of a salt with a mineral acid, e.g., hydrochloric acid, hydrobromic acid, or sulfuric acid. The primary amines and compounds of Formula 8 are commercially available or easily prepared as described in the literature, e.g., Capuano et al. (1971), Chem. Ber. *104*, 3039; Robins et al. (1974), J. Het. Chem. *11*, 199; Bel patent 857,388 (1978); Bel. Patent 875,846 (1979); Bel Patent 885,089 (1981).

Specific compounds are made as follows.

*2-(4-imidazolylethylamino)-4-oxo-1H,3H-pyrazolo(1,5-a)-1,3,5-triazine*

A mixture of 135 mg of free histamine and 180 mg of 2-methylthio-4-oxo-3H-pyrazolo(1,5-a)-1,3,5-triazine in 10 ml of xylene is refluxed for 22 hrs. After evaporation of the solvent *in vacuo*, the pale pink residue is triturated with water and alcohol, filtered, washed with ether and dried to yield 120 mg of purified product. TLC (silica gel $CHCl_3$/MeOH/triethylamine = 3:1:0—5) Rf = 0.14.

*4-(2-(5-methyl-4-imidazolylmethylthio)-ethylamino)-2-methylthiopyrazolo[1,5-a]-1,3,5-triazine*

500 mg of 4-oxo-2-thioxo-1,2,3,4-tetrahydro-s-triazolo(2,3-a)-5-triazine is dissolved in 3.4 ml of 1.73N NaOH. The solution is diluted with 12 ml methanol, treated with 0.184 ml methyliodide, and stirred at room temperature for $\frac{1}{2}$ hr. The white sodium salt is collected by filtration, redissolved in water, and acidified with $H_2SO_4$. The precipitate is collected by filtration and dried.

100 mg of the resulting 4-oxo-2-methylthio-1,2,3,4-tetrahydro-s-triazolo(2,3-a)-5-triazine are suspended in 1.5 ml of $POCl_3$. Two drops of N,N-dimethylaniline are added, and the mixture is refluxed for 3½ hrs. The $POCl_3$ is removed *in vacuo*, and the residue treated with ice and $CHCl_3$. The chloroform layer is washed with water several times and dried. 4-chloro-2-methylthio-1,2,3,4-tetrahydro-s-triazolo(2,3-a)-5-triazine is recovered from the chloroform and chromatographed on silica gel using chloroform as the eluant.

The final product of this synthesis is produced by treating with 77 mg of free histamine a solution of 70 mg 4-chloro-2-methylthio-1,2,3,4-tetrahydro-s-triazolo(2,3-a)-5-triazine in 5 ml methanol. The mixture is stirred at room temperature overnight. The yellow precipitate is collected and recrystallized from methanol. Additional product is retrieved from the filtrate after it is concentrated *in vacuo*. TLC (Silica gel: $CHCl_3$/$CH_3OH$ = 3:1) Rf = 0.43.

*8-bromo-4-(2-(5-methyl-4-imidazolylmethylthio)-ethylamino)-2-methylthiopyrazolo[1,5-a]-1,3,5-triazine*

8 - bromo - 4 - (2 - (5 - methyl - 4 - imidazolylmethylthio) - ethylamino) - 2 - methylthiopyrazolo-[1,5-a] - 1,3,5 - triazine is prepared from 8-bromo-4-chloro-2-methylthiopyrazolo[1,5-a]-1,3,5-triazine according to conventional methods.

*4-(4-Imidazolylethylamino)-2-methylthiopyrazolo(1,5-a)-1,3,5-Triazine*

A solution of 30 mg 4-chloro-2-methylthiopyrazolo(1,5-a)-1,3,5-triazine and 33 mg free histamine in 3 ml of methanol is stirred at room temperature overnight. After evaporation of solvent, the residue is dissolved in ethylacetate, washed with water, and dried over $MgSO_4$. After removal of solvent, the residue is subject to silica gel preparative thin layer chromatography using $CHCl_3$/methanol = 9:1 as a developing solvent. Appropriate fractions are isolated, extracted with $CHCl_3$/$CH_3OH$ (3:1), and solvent removed *in vacuo* to dryness. 20 mg of a white solid are recovered. TLC (silica gel $CHCl_3$/$CH_3OH$ = 3:1) Rf = 0.15.

*8-bromo-4-(4-imidazolylethylamino)-2-methylthiopyrazolo-[1,5-a]-1,3,5-triazine*

8-bromo-4-(4-imidazolylethylamino)-2-methylthiopyrazolo-[1,5-a]-1,3,5-triazine is prepared from 8-bromo-4-chloro-2-methylthiopyrazolo[1,5-a]-1,3,5-triazine according to conventional methods.

*Use*

When administered to mammals (e.g. orally, topically, intravenously, parenterally, nasally, or by suppository), the compounds of the invention can reduce gastric acid secretion.

This action can render the compounds useful in the treatment of peptic, gastric, or duodenal ulcers, Zollinger-Ellison syndrome (gastrinoma), systemic mastocytosis, basophil leukemia associated with hyper-histamenia, short bowel syndrome, reflux esophagitis, acute erosive gastritis, and pancreatic insufficiency. The compounds can also be useful in the treatment of some cancers.

The compounds can be administered to a mammal in a dosage of 2 to 10 mg/kg/day, preferably 4 to 8 mg/kg/day.

**Claims**

1. A compound having gastric acid secretion reducing activity and having the general formula

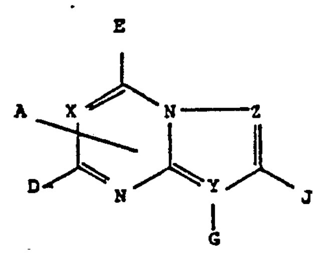

(1)

wherein D is H, SH, OH, $R^4S$ where $R^4$ is lower alkyl group having fewer than 6 carbon atoms, or $NH_2$; E is OH or $NH_2$; J is H or single aryl ring; X is CH or N; Y is CH or N or CT, wherein T is a halogen; Z is CH or N; A is

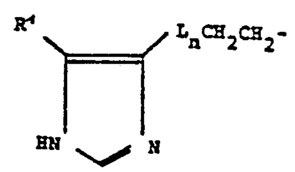

or

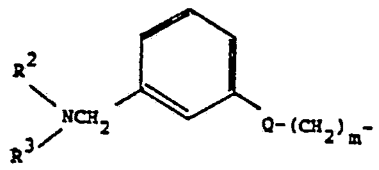

or

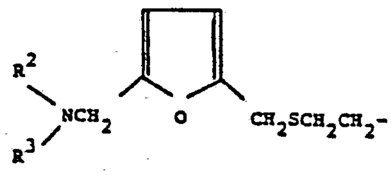

and replaces a hydrogen of either D or E; $R^1$ is H or $CH_3$; L is $CH_2S$; Q is O or $CH_2S$; n is 0 or 1; $2 \leq m \leq 4$; each $R^2$ and $R^3$, independently, is H, lower alkyl, a cycloalkyl group having fewer than 6 carbon atoms, or an aryl group with, optionally, an alkyl group attached having fewer than 6 carbon atoms; or $R^2$ and $R^3$, together with the nitrogen atom to which they are attached, form a 4, 5, or 6 membered heterocyclic ring containing 0, 1, or 2 oxygen atoms and 1, 2, or 3 nitrogen atoms and being unsubstituted or lower alkyl having fewer than 6 carbon atoms substituted; or a pharmaceutically acceptable salt thereof.

2. The compound of claim 1, wherein A is

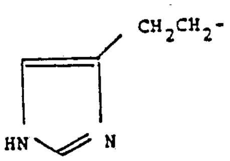

and is attached to D; D is NH; E is OH; G is H; J is H; X is N; Y is C; and Z is N; said compound having the name 2-(4-imidazolyl-ethylamino)-4-oxo-1H,3H-pyrazolo[1,5-a]-1,3,5-triazine; or a pharmaceutically acceptable salt thereof.

3. The compound of claim 1, wherein A is

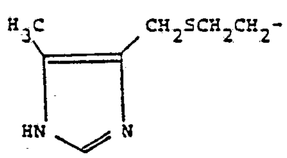

and is attached to E; D is $SCH_3$; E is NH; G is H; J is H; X is N; Y is C; and Z is N; said compound having the name 4-(2-(5-methyl-4-imidazolylmethylthio)-ethylamino)-2-methylthiopyrazolo[1,5-a]-1,3,5-triazine; or a pharmaceutically acceptable salt thereof.

4. The compound of claim 1, wherein A is

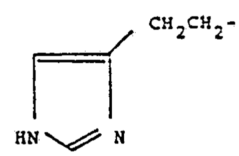

and is attached to E; D is $CH_3S$; E is NH; G is H; J is H; X is N; Y is C; and Z is N; said compound having the name 4-(4-imidazolyl-ethylamino)-2-methylthiopyrazolo[1,5-a]-1,3,5-triazine; or a pharmaceutically acceptable salt thereof.

5. The compound of claim 1, wherein A is

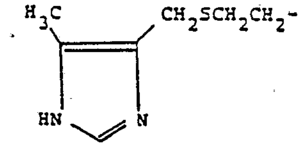

and is attached to E; D is $SCH_3$; E is NH; G is Br; J is H; X is N; Y is C; and Z is N; said compound having the name 8-bromo-4-(2-(5-methyl-4-imidazolylmethylthio)-ethylamino)-2-methylthiopyrazolo[1,5-a]-1,3,5-triazine; or a pharmaceutically acceptable salt thereof.

6. The compound of claim 1, wherein A is

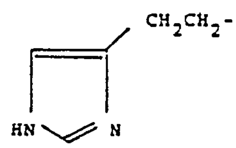

and is attached to E; D is $CH_3S$; E is NH; G is Br; J is H; X is N; Y is C; and Z is N; said compound having the name 8-bromo-4-(4-imidazolylethylamino)-2-methylthiopyrazolo[1,5-a]-1,3,5-triazine; or a pharmaceutically acceptable salt thereof.

7. A therapeutic composition comprising a therapeutically effective amount of the compound of claim 1 together with a pharmaceutically acceptable carrier substance.

8. The therapeutic composition of claim 7, wherein said composition is in the form of a tablet, capsule, or for oral administration to a human patient in need of said compound.

9. A gastric secretion reducing amount of the compound of claim 1, 2, 3, 4, 5, or 6, for treating a mammal suffering from gastric hypersecretion.

**Patentansprüche**

1. Zusammensetzung zur Magensäuresekretions-Verringerung mit der Formel

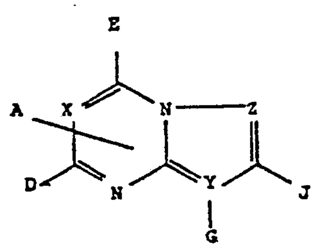

(1)

wobei

D = H, SH, OH, $R^4S$, $R^4$ = niedrige Alkylgruppe mit unter 6 Kohlenstoff-Atomen oder $NH_2$
E = OH oder $NH_2$;
G = H oder ein Halogen
J = H oder einzelner Aryl-Ring
X = CH oder N;
Y = CH oder N oder CT mit T = ein Halogen
Z = CH oder N
A =

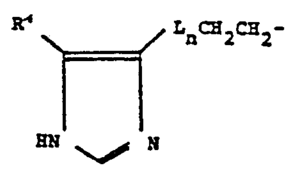

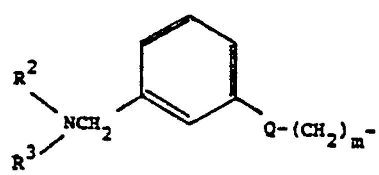

oder

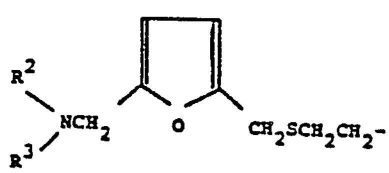

und einen Wasserstoff von entweder D oder E ersetzt;
$R^1$ = H oder $CH_3$
L = $CH_2S$
Q = O oder $CH_2S$

n = 0 oder 1

$2 \leq m \leq 4$

$R^2$, $R^3$ = jeweils (unabhängig) H, niedriges Alkyl, eine Cycloalkyl-Gruppe mit unter 6 Kohlenstoff-Atomen oder eine Aryl-Gruppe mit — wahlweise — einer gebundenen Alkyl-Gruppe mit unter 6 Kohlenstoff-Atomen

oder

$R^2$, $R^3$ zusammen mit dem Wasserstoff-Atom, an das sie gebunden sind, einen 4-, 5- oder 6-gliedrigen heterocyclischen Ring bilden, der 0, 1 oder 2 Sauerstoff-Atome enthält und unsubstituiert oder substituiert ist durch niedriges Alkyl mit unter 6 Kohlenstoff-Atomen,

oder pharmazeutisch verträgliches Salz daraus.

2. Zusammensetzung nach Anspruch 1 mit

A =

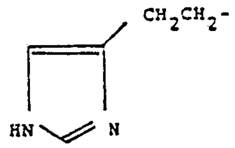

und gebunden an D;

D = NH;

E = OH;

G = H;

J = H;

X = N;

Y = C;

Z = N; und

dem Namen 2-(4-Imidazolyl-Ethylamino)-4-Oxo-1H,3H-Pyrazolo[1,5-a]-1,3,5-Triazin;

oder ein pharmazeutisch verträgliches Salz davon.

3. Zusammensetzung nach Anspruch 1 mit

A =

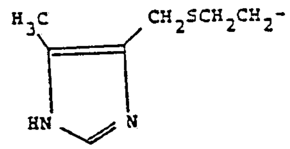

und gebunden an E;

$D = SCH_3$;

E = NH;

G = H;

J = H;

X = N;

Y = C;

Z = N; und

dem Namen 4-(2-(5-Methyl-4-Imidazolylmethylthio)-Ethylamino)-2-Methylthiopyrazolo[1,5-a]-1,3,5-Triazin;

oder ein pharmazeutisch verträgliches Salz davon.

4. Zusammensetzung nach Anspruch 1 mit

A =

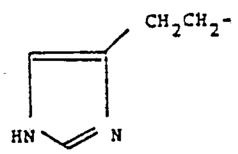

und gebunden an E;

$D = CH_3S$;

E = NH;

G = H;

J = H;

X = N;

Y = C;

Z = N; und

dem Namen 4-(4-Imidazolyl-Ethylamino)-2-Methylthiopyrazolo[1,5-a]-1,3,5-Triazin;

oder ein pharmazeutisch verträgliches Salz davon.

5. Zusammensetzung nach Anspruch 1 mit

A =

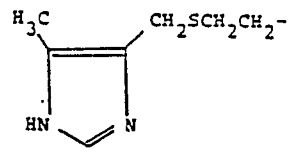

und gebunden an E;

D = $SCH_3$;

E = NH;

G = Br;

J = H;

X = N;

Y = C;

Z = N; und

dem Namen 8-Bromo-4-(2-(5-Methyl-4-Imidazolylmethylthio)-Ethylamino)-2-Methylthiopyrazolo[1,5-a]-1,3,5-Triazin;

oder ein pharmazeutisch verträgliches Salz davon.

6. Zusammensetzung nach Anspruch 1 mit

A =

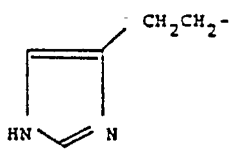

und gebunden an E;

D = $CH_3S$;

E = NH;

G = Br;

J = H;

X = N;

Y = C;

Z = N; und

dem Namen 8-Bromo-4-(4-Imidazolylethylamino)-2-Methylthiopyrazolo[1,5-a]-1,3,5-Triazin;

oder ein pharmazeutisch verträgliches Salz davon.

7. Therapeutische Zusammensetzung mit therapeutisch wirksamer Menge der Zusammensetzung nach Anspruch 1 zusammen mit pharmazeutisch verträglicher Trägersubstanz.

8. Therapeutische Zusammensetzung nach Anspruch 7 in Form einer Tablette oder Kapsel zur oralen Verabreichung an Menschen.

9. Magensaftsekretion vermindernde Menge der Zusammensetzung nach einem der Ansprüche 1—6 zur Behandlung eines Säugers gegen gastritische Hypersekretion.

**Revendications**

1. Composé présentant une activité réductrice de la sécrétion acide gastrique et présentant la formule

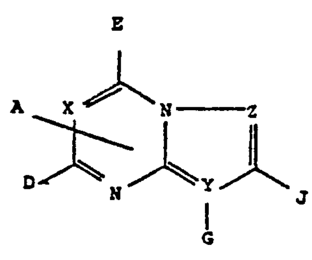

(1)

dans laquelle D représente H, SH, OH, $R^4S$ où $R^4$ est un groupe alcoyle inférieur comportant moins de 6 atomes de carbone, ou $NH_2$; E représente OH ou $NH_2$; J représente H ou un cycle aryle unique; X représente CH ou N; Y représente CH ou N ou CT dans laquelle T représente un halogène; Z représente CH ou N; A représente

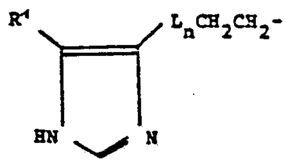

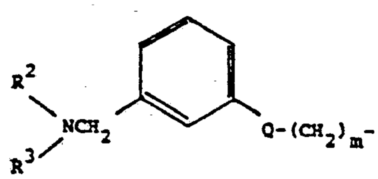

ou

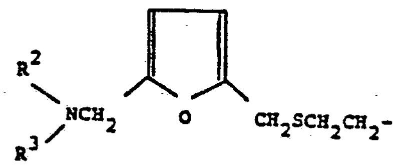

et remplace un hydrogène, soit de D, soit de E; $R^1$ représente H ou $CH_3$; L représente $CH_2S$; Q représente O ou $CH_2S$; n est 0 ou 1; $2 \leq m \leq 4$; chacun des substituants $R^2$ et $R^3$, indépendamment, représente H, un alcoyle inférieur, un groupe cycloalcoyle ayant moins de 6 atomes de carbone ou un groupe aryle avec au choix un groupe alcoyle rattaché ayant moins de 6 atomes de carbone; ou $R^2$ et $R^3$, conjointement avec l'atome d'azote sur lequel ils sont fixés, forment un cycle hétérocyclique à 4, 5 ou 6 éléments contenant 0, 1 ou 2 atomes d'oxygène et 1, 2 ou 3 atomes d'azote et étant non substitués ou un alcoyle inférieur comportant moins de 6 atomes de carbone substitués; ou un de leurs sels acceptables pharmaceutiquement.

2. Composé selon la revendication 1, dans lequel A représente

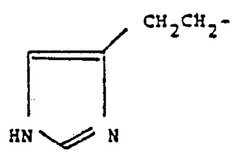

et est fixé sur D; D représente NH; E représente OH; G représente H; J représente H; X représente N; Y représente C; et Z représente N; ledit composé étant désigné par 2-(4-imidazolyl-éthylamino)-4-oxo-1H,3H-pyrazolo-[1,5-a]-1,3,5-triazine; ou un de ses sels acceptables pharmaceutiquement.

3. Composé selon la revendication 1, dans lequel A représente

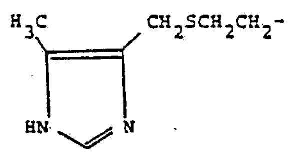

et est fixé sur E; D représente $SCH_3$; E représente NH; G représente H; J représente H; X représente N; Y représente C; et Z représente N; ledit composé étant désigné par 4-(2-(5-méthyl-4-imidazolylméthylthio)-éthylamino)-2-méthylthiopyrazolo-[1,5-a]-1,3,5-triazine; ou un de ses sels acceptables pharmaceutiquement.

4. Composé selon la revendication 1, dans lequel A représente

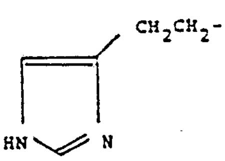

et est fixé sur E; D représente $CH_3S$; E représente NH; G représente H; J représente H; X représente N; Y représente C; et Z représente N; ledit composé étant désigné par 4-(4-imidazolyl-éthylamino)-2-méthylthio-pyrazolo-[1,5-a]-1,3,5-triazine; ou un de ses sels acceptables pharmaceutiquement.

5. Composé selon la revendication 1, dans lequel A représente

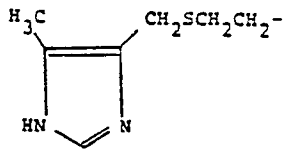

et est fixé sur E; D représente $SCH_3$; E représente NH; G représente Br; J représente H; X représente N; Y représente C; et Z représente N; ledit composé étant désigné par 8-bromo-4-(2-(5-méthyl-4-imidazolyl-méthylthio)-éthylamino)-2-méthylthiopyrazolo-[1,5-a]-1,3,5-triazine; ou un de ses sels acceptables pharmaceutiquement.

6. Composé selon la revendication 1, dans lequel A représente

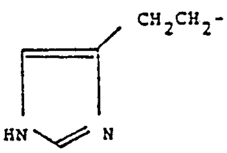

et est fixé sur E; D représente $SH_3S$; E représente NH; G représente Br; J représente H; X représente N; Y représente C; et Z représente N; ledit composé étant désigné par 8-bromo-4-(4-imidazolyléthylamino)-2-méthylthiopyrazolo-[1,5-a]-1,3,5-triazine; ou un de ses sels acceptables pharmaceutiquement.

7. Composition thérapeutique comprenant une quantité efficace thérapeutiquement du composé de la revendication 1 conjointement avec un excipient acceptable pharmaceutiquement.

8. Composition thérapeutique selon la revendication 7, dans laquelle la composition est sous la forme d'un comprimé d'une capsule ou destinée à l'administration orale chez un patient humain nécessitant ce composé.

9. Quantité réductrice de la sécrétion gastrique du composé de la revendication 1, 2, 3, 4, 5 ou 6, pour traiter un mammifère souffrant d'hypersécrétion gastrique.